(19) Europäisches Patentamt
European Patent Office
Office européen des brevets

(11) **EP 3 653 248 B1**

(12) **EUROPÄISCHE PATENTSCHRIFT**

(45) Veröffentlichungstag und Bekanntmachung des Hinweises auf die Patenterteilung:
**21.12.2022   Patentblatt 2022/51**

(21) Anmeldenummer: **20150286.1**

(22) Anmeldetag: **10.03.2016**

(51) Internationale Patentklassifikation (IPC):
**A61M 16/12** *(2006.01)*   **A61M 16/04** *(2006.01)*
**A61M 16/00** *(2006.01)*   **A61B 5/08** *(2006.01)*
**A61B 5/091** *(2006.01)*

(52) Gemeinsame Patentklassifikation (CPC):
(C-Sets verfügbar)
**A61B 5/091; A61B 5/4836; A61M 16/0066;
A61M 16/026; A61M 16/12; A61M 16/202;
A61M 16/204; A61M 16/205;** A61B 5/0826;
A61M 16/04; A61M 2016/0027; A61M 2016/0036;
A61M 2016/0039; A61M 2016/0042;
A61M 2202/0208;                                   (Forts.)

(54) **BEATMUNGSGERÄTE**

RESPIRATORS

APPAREILS RESPIRATOIRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.03.2015   DE 102015103894**

(43) Veröffentlichungstag der Anmeldung:
**20.05.2020   Patentblatt 2020/21**

(60) Teilanmeldung:
**22203061.1**

(62) Dokumentnummer(n) der früheren Anmeldung(en) nach Art. 76 EPÜ:
**16717238.6 / 3 270 993**

(73) Patentinhaber: **Fritz Stephan GmbH
56412 Gackenbach (DE)**

(72) Erfinder:
 • **Braun, Wolfgang
   56428 Dernbach (DE)**
 • **Herber-Jonat, Susanne
   82234 Weßling (DE)**
 • **Höhne, Bernd
   56130 Bad Ems (DE)**
 • **Hummler, Helmut
   89081 Ulm (DE)**
 • **Schulze, Andreas
   82432 Walchensee (DE)**

(74) Vertreter: **Hellmich, Wolfgang
   European Patent and Trademark Attorney
   Lortzingstrasse 9 / 2. Stock
   81241 München (DE)**

(56) Entgegenhaltungen:
   WO-A2-2008/058417      US-A1- 2009 320 836
   US-A1- 2011 041 849      US-A1- 2012 071 729

 • **HERBER-JONAT SUSANNE ET AL: "Adaptive mechanical backup ventilation for preterm infants on respiratory assist modes - a pilot study", INTENSIVE CARE MEDICINE, BERLIN, DE, Bd. 32, Nr. 2, 24. Januar 2006 (2006-01-24), Seiten 302-308, XP037119511, ISSN: 0342-4642, DOI: 10.1007/S00134-005-0003-7 [gefunden am 2006-01-24]**
 • **SCHULZE ET AL: "Enhancement of mechanical ventilation of neonates by computer technology", SEMINARS IN PERINATOLOGY, W.B. SAUNDERS, GB, Bd. 24, Nr. 6, 1. Dezember 2000 (2000-12-01), Seiten 429-444, XP005451811, ISSN: 0146-0005, DOI: 10.1053/SPER.2000.20084**

(52) Gemeinsame Patentklassifikation (CPC): (Forts.)
A61M 2205/3331; A61M 2205/502; A61M 2230/205;
A61M 2230/42; A61M 2230/60; A61M 2240/00

C-Sets
A61M 2230/205, A61M 2230/005;
A61M 2230/60, A61M 2230/005

**Beschreibung**

Das Gebiet der Erfindung sind Beatmungsgeräte der im Oberbegriff des Patentanspruchs 1 genannten Art. Solche Beatmungsgeräte sind aus der US 2011/041849 A1 bekannt.

Die Erfindung bezieht sich insbesondere auf Beatmungsgeräte, die die durch ein Oxymeter gemessene Sauerstoffsättigung im Blut berücksichtigen.

Beatmungsgeräte zur maschinellen künstlichen Beatmung bei allen Formen des Sauerstoffmangelzustands sind bekannt (Roche Lexikon Medizin, 4. Auflage, Urban & Fischer Verlag, München). Solche Beatmungsgeräte werden auch als Respiratoren bezeichnet. Dabei wird zwischen drei Grundtypen unterschieden:

a) druckgesteuerte Beatmungsgeräte: die Inspirationsphase ist beendet, wenn im Gerät ein vorgegebener Beatmungsdruck erreicht ist. Die Exspiration erfolgt meist passiv.

b) volumengesteuerte Beatmungsgeräte: die Inspiration ist beendet, wenn ein vorher eingestelltes Gasvolumen den Respirator verlassen hat. Die Exspiration erfolgt meist passiv.

c) zeitgesteuerte Beatmungsgeräte: das Gasgemisch wird innerhalb einer vorher eingegebenen Zeit abgegeben.

Beatmungsgeräte neueren Typs verfügen über elektronische Steuerungen, die patientengerechte Beatmungstypen erlauben. Solche Beatmungsgeräte weisen häufig einen Drucksensor auf, mit dem der Beatmungsdruck gemessen wird. Der Drucksensor kann sich im Beatmungsgerät befinden und so den Druck am beatmungsgerätseitigen Ende des Beatmungsschlauches messen.

Zusätzlich dürften die meisten elektronisch gesteuerten Beatmungsgeräte auch einen Flusssensor zur Messung des Luftflusses $\dot{V}$, also des pro Zeiteinheit ein- oder ausgeatmetem Luftvolumens aufweisen. Ein solcher Flusssensor wird auch als Spirometer bezeichnet. Durch Aufsummieren der vom Flusssensor gelieferten Flusswerte während einer Inspirations- oder Exspirationsphase erhält man Inspirations- bzw. Exspirationsvolumina. Mathematisch etwas feiner ausgedrückt ergibt sich ein Inspirations- oder Exspirationsvolumen als Integral über den Atemfluss $\dot{V}$ über die Zeit einer Inspirations- bzw. Exspirationsphase

EP 2 091 429 B1 offenbart eine Vorrichtung, die so ausgerüstet ist, dass sie den PEEP bestimmen kann. Sie besitzt einen Fluss/Drucksensor, mit dem Druck und Volumen gemessen werden können. Dieser ist z.B. patientenseitig an einem Beatmungsschlauch angeordnet oder im Gerät integriert. Durch diesen Beatmungsschlauch wird der Patient mittels des Beatmungsgeräts beatmet. Wenn man das Atemvolumen V über dem Beatmungsdruck p aufträgt, wird diese so genannte P/V-Schleife eine Hysterese auf. Der untere, aufsteigende Ast Vinf (inf wie inflation) wird aufgezeichnet, während die Lunge aufgebläht wird (Inspiration). Der obere, absteigende Ast Vdef (def wie deflation) wird erfasst während Luft aus der Lunge entweicht (Exspiration). Der angemessene PEEP lässt sich in einem Diagramm an der Schlaufe ablesen. Der angemessen PEEP ist der Druck, bei dem die größte Volumendifferenz zwischen Vdef und Vinf gemessen wird. Weil Inspirations- und Exspirationsvolumen in EP 2 091 429 B1 gleich sind, weil sich die Äste Vinf und Vdef rechts oben und links unten treffen, also eine geschlossene P/V-Schleife bilden, kann es in EP 2 091 429 B1 kein EFE-Ereignis (s.u.) geben.

US 2012/0071729 A1 beschreibt ein übliches Beatmungsgerät für einen menschlichen Patienten. Das Beatmungsgerät umfasst ein pneumatische System zur Druckerzeugung und ist über ein Schlauchsystem und eine physikalische Patientenschnittstelle mit dem Patienten verbunden. Das Beatmungsgerät umfasst unter anderem eine Steuerung und ein Oxymeter. Unterschiedliche Alarme können ausgelöst werden. Falls $SpO_2$, PEEP, oder $FiO_2$ eine vorbestimmte Schwelle über- oder unterschreiten, kann ein Alarm ausgelöst werden. Falls der PEEP beispielsweise von einer Bedienungsperson herabgesetzt wurde, bevor die $SpO_2$ einen Schwellenwert unterschreitet, wird ein anderer Alarm ausgelöst. Falls die $FiO_2$ herabgesetzt wurde, bevor die $SpO_2$ einen Schwellenwert unterschreitet, wird ein dritter Alarm ausgelöst. US 2012/0071729 A1 beschreibt nicht, dass der PEEP irgendwie geändert wird, insbesondere nicht, den PEEP zu erhöhen, wenn die $SpO_2$ unter einen Grenzwert fällt.

In Kenntnis des Luftflusses lässt sich der Druckabfall am Beatmungsschlauch (vgl. EP 1 562 655 B1) und/oder einem eventuell vorhandenen Endotrachealtubus oder einer Trachealkanüle berechnen. Natürlich lässt sich der Druckabfall auch an Teilen des Beatmungsschlauchs und/oder des Endotrachealtubus oder der Trachealkanüle berechnen. So lässt sich für die Regelung auf den Druck ein beliebiger Punkt zwischen dem Beatmungsgerät und der Lunge abstellen. Sinnvoll erscheint es den Druck im Mund zu wählen, weil dieser am ehesten dem Lungendruck entspricht. In den neueren Beatmungsgeräten sind die verfügbaren Beatmungsformen im Allgemeinen Kombinationen der eingangs aufgeführten drei Grundtypen.

Die CPAP-Beatmung (Continuous Positive Airway Pressure) ist eine Beatmungsform, die die Spontanatmung des Patienten mit einem dauerhaften Überdruck von typischerweise 5 bis 30 mbar kombiniert. Der Patient kann seine Atemtiefe, Atemfrequenz und auch den Atemfluss $\dot{V}$ selbst bestimmen. Wie man Atemzüge unter CPAP erkennt, ist beispielsweise in der EP 1 294 428 B1 beschrieben.

IPPV (Intermittent Positive Pressure Ventilation, deutsch: Beatmung mit pulsierend positivem Druck) wird in der Intensiv- und Notfallmedizin eingesetzt und bezeichnet eine volumenkontrollierte Form der Beatmung mit einem Beatmungsgerät. Synonym zur Abkürzung IPPV wird heute häufiger der Begriff Volume Controlled Ventilation verwendet und mit VCV abgekürzt. Bei einer volumenkontrollierten Beatmung versucht das Beatmungsgerät das Volumen konstant zu halten und variiert dafür den Beatmungsdruck. Bei dieser Beatmungsform kann der Luftdruck in den Atemwegen am Ende der Ausatmung (Exspiration) auf bis zu 0 mbar absinken, erreicht jedoch keine negativen Werte.

Häufiger wird heute die druckkontrollierte Beatmungsform genutzt. Sie gibt zwei unterschiedliche Druckniveaus vor: eines für die Einatmung und eines für die Ausatmung. Man nennt diese Beatmungsform auch Bilevel oder BIPAP (Bi Positive Airway Pressure, deutsch: 2 Atemwegsüberdruck). Bei BIPAP herrscht auch am Ende der Exspiration noch ein Überdruck (PEEP, Positive End-Exspiratory Pressure) in den Atemwegen.

Laut Rathgeber, Grundlagen der maschinellen Beatmung, ISBN 9783131487926, 2010, Georg Thieme Verlag KG, werden bei der volumenkontrollierten Beatmung sämtliche Beatmungsparameter vorgegeben. Ziel- und Steuerungsparameter ist das Tidalvolumen (Atemzugvolumen). Die resultierenden Atemwegsdrücke sind abhängig von den eingestellten Volumina sowie den pulmonalen Gegebenheiten des Patienten. Die Beeinflussung des inspiratorischen Beatmungsmusters durch den Patienten ist nicht möglich. Typischerweise können bei einer volumenkontrollierten Beatmung vier Parameter vorgegeben werden, nämlich die Höhe des Inspirationsflusses, das Atemminutenvolumen, die Beatmungsfrequenz und der Druck am Ende der Exspiration (PEEP). Nach Öffnung des Inspirationsventils wird bis zum Ende der Inspirationshase ein konstanter Fluss definierter Höhe abgegeben. Andere Flussmuster, wie dezelerierender, akzelerierender oder sinusförmiger Fluss werden bei volumenkontrollierter Beatmung praktisch nicht mehr verwendet, da sie keine erkennbaren Vorteile bieten. Die primäre Einstellung des Atemminutenvolumens und damit des Inspirationsflusses, orientiert sich am Körpergewicht des Patienten, wobei ca. 250 ml Atemluft pro kg Körpergewicht und Minute bei Neugeborenen zugrunde gelegt wird. Die initiale Beatmungsfrequenz wird bei Neugeborenen auf ca. 50 pro Minute eingestellt.

Die IPPV findet man überwiegend im Bereich des Rettungsdienstes, da auf den hier vorgehaltenen Fahrzeugen nur Notfallrespiratoren eingesetzt werden, welche meist keine anderen Beatmungsformen unterstützen.

Bei der S-IPPV (Synchronized Intermittent Positive Pressure Ventilation) handelt es sich um eine synchronisierte IPPV. Hier werden Atembemühungen des Patienten ("Trigger") erkannt und synchronisiert. Daneben gibt es die SNIPPV, wobei das N für non-invasive oder nicht-invasiv, also für Verwendung einer Beatmungsmaske, einer Nasenbrille oder einem ähnlichen Patienteninterface ohne Trachealkanüle oder Endotrachealtubus steht.

PAV (Proportional Assist Ventilation) ist eine Beatmungsform, in der das Beatmungsgerät einen bestimmten Anteil der für die Atemtätigkeit notwendigen Arbeit übernimmt. Das Beatmungsgerät verändert das Atemvolumen und den Druck in Abhängigkeit der Atemtätigkeit des Patienten. Je stärker der Patient atmet, desto mehr Atemvolumen und Druck liefert das Beatmungsgerät.

Bei Unreifgeborenen gibt es einen Reflex, die Atemtätigkeit einzustellen, wenn die Sauerstoffsättigung zu tief ist. Die Kontrolle der Sauerstoffsättigung $SpO_2$ des Blutes durch Veränderung der Sauerstoffkonzentration des dem Patienten zugeführten Atemgases $FiO_2$ ist eine im Stand der Technik beschriebene Vorgehensweise bei der Beatmung von Patienten mit Atemstörungen. Das Verfahren ist z.B. in "NEONATAL WORKSTATION FOR INSPIRED OXYGEN CONTROL AND CLINICAL MONITORING" von Yao Sun et al., 1994, AAAI Technical Report SS-94-01 beschrieben. Ein ähnliches Verfahren ist in WO 02/47741 A2 beschrieben.

In "Adaptive mechanical backup ventilation for preterm infants on respiratory assist modes" von Susanne Herber-Jonat et al., 2006, Intensive Care Med 32:302-308, Springer, ist beschrieben, dass eine PAV im Fall von Apnoen, also Atmungsaussetzer, oder zu geringer Atmung durch eine Zwangsbeatmung ("mechanical backup") ergänzt wird. Hierzu kann (1) der Arzt eine Zeitspanne zwischen dem Ende der Atemtätigkeit und dem automatischen Beginn der Zwangsbeatmung einstellen. (2) Die Zwangsbeatmung wird schrittweise reduziert, in dem die Frequenz der Zwangsbeatmung während der Wiederaufnahme der spontanen Atmung reduziert wird. (3) Spontanatmung ohne Zwangsbeatmung wird nur dann unter CPAP oder PAV weiterhin erlaubt, wenn die Sauerstoffsättigung $SpO_2$ des Blutes einen benutzerdefinierten Schwellenwert überschreitet. Dieser Zwangsbeatmungsmodus wird als $SpO_2$-empfindliche, adaptive Zwangsbeatmung ("$SpO_2$-sensitive adaptive backup") bezeichnet. Es wurde klinisch gezeigt, dass ein ausschleichendes Backup die Therapie verbessert. Ferner ist es das Ziel einer Therapie die Unterstützung so gering wie möglich zu halten, da jede Form der Beatmung potentiell schädlich ist.

Die EP 2 671 509 A1 offenbart ein Beatmungssystem, bei dem die Atmungtätigkeit eines Patienten mittels eines Respirations-Sensorelements erfasst wird. Das Sensorelement besteht aus einem Sensorteil, welches direkt auf die Haut seitlich am Abdomen (Bauch) des Patienten appliziert wird, und aus einem Kabelteil, mittels welchem von dem Sensorteil erzeugte Signale an eine Datenschnittstelle einer Elektronikeinrichtung des Beatmungssystems übertragen werden können. Die erzeugten Signale werden durch Bilden eines arithmetischen Mittelwertes in Bezug auf zuvor erzeugte Signale korrigiert und so kalibriert. Für den Fall, dass eine solche rechnerische Kalibrierung ausgeschlossen ist, umfasst das Beatmungssystem Kalibrierungsmittel zur Kalibrierung des mit dem Patienten wechselwirkenden Sensorelements. Die Kalibrierungsmittel umfassen eine Einrichtung zum Betätigen eines Ventilelements zur Belüftung und/oder Entlüftung des Beatmungssystems.

Die US 2011/041849 A1 offenbart ein Beatmungssystem mit einem Beatmungsgerät, das über ein Kabel mit einem Oxymetergerät verbunden ist, das eine Vielzahl von Pulsoxymetern mit einem oder mehreren Sensoren umfasst. Das Beatmungsgerät umfasst eine $FiO_2$-Steuerung, die einen Prozessor zur Ausführung von Software und einen Speicher, der ein RAM oder ROM sein kann, umfasst. Das Beatmungssystem kann eine Anzeige umfassen, die mit dem Beatmungsgerät über ein Kabel verbunden ist, das mit einer digitalen Kommunikationsschnittstelle verbunden ist. Insbesondere unter Verwendung von Schwellwerten, aber auch Durchschnitten wird aus 2 Sauerstoffsättigungswerten eine Sauerstoffsättigungsmetrik berechnet. Sobald eine Sauerstoffsättigungsmetrik berechnet ist, kann eine Beatmungsgeräteinstellung unter Benutzung der Sauerstoffsättigungsmetrik bestimmt werden. Beispielsweise kann die Beatmungsunterstützung erhöht werden, wenn die Sauerstoffsättigungsmetrik eine geringe Sauerstoffsättigung angibt und umgekehrt. Die Einstellungen am Beatmungsgerät können unter anderem den Anteil des eingeatmeten Sauerstoffs $FiO_2$, das Atemvolumen, den maximalen Atemfluss beim Einatmen, den PEEP oder eine Kombination der Einstellungen umfassen.

Die US 2009/320836 A1 beschreibt ein medizintechnisches Gerät mit einer Hauptsteuerung, einem Beatmungsgerät, einem Sensor, beispielsweise einem Pulsoxymetersensor für einen Patienten. Gas aus einer Luftquelle, einer Sauerstoffversorgung sowie einer Stickstoffversorgung wird über Ventile und Flusssensoren zusammengeführt und dem Patienten über eine Inspirationsleitung und ein Applikationsstück zugeführt. Einer Hauptsteuerung innerhalb einer Primärsteuerung werden Messwerte von Sensoren, beispielsweise Pulsoxymetern, zugeführt. Die Hauptsteuerung steuert über Steuerungen die sich ebenfalls in der Primärsteuerung befinden, die Ventile. Daneben gibt es eine Exspirationsleitung. Die Primärsteuerung kann den PEEP basierend auf der Sauerstoffbehandlung des Patienten steuern. Die Primärsteuerung kann einen geschlossenen Schleifen-$FiO_2$-Regler enthalten, der die $FiO_2$ um einen Wert erhöht oder erniedrigt, der vom Unterschied zwischen einem gemessenen und einem Ziel-$SpO_2$ abhängt. Falls der $SpO_2$ über einem Zielwert liegt, wird der $FiO_2$ durch eine Erhöhung des Stickstoffflusses und eine Reduzierung des Luft- und Sauerstoffflusses reduziert. Das System kann den PEEP basierend auf der Sauerstoffbehandlung des Patienten einstellen. Der PEEP kann abgesenkt werden, falls die Therapie über ein gewisses Niveau eingestellt werden musste.

Die in der WO 2008/058417 A1, Figur 1 dargestellte Ausführungsform umfasst ein Beatmungsgerät das eine einzige Regelschleife entweder für den Atemwegsdruck oder den Luftfluss umfasst. Im CPAP-Modus gibt der Benutzer einen einzigen Wert für den Sollüberdruck in den Atemwegen ein. Das Signal des Istatemwegsdrucks wird zurückgekoppelt. Um eine druckgesteuerte zyklische Beatmung zu realisieren, kann der gleiche Aufbau verwendet werden. Jedoch ändert ein Funktionsgenerator den Sollüberdruck in den Atemwegen zwischen dem Überdruck am Ende der Exspiration (PEEP) und dem maximalen Inspirationsdruck (PIP). Um eine volumengesteuerte, zyklische Beatmung zu erzeugen, wird der Luftfluss während der Inspiration in der Regelschleife mit einem Sollfluss als Zielwert benutzt. Während der Exspiration kehrt das System zur Drucksteuerung zurück, wobei der Sollwert der PEEP ist.

Die in der WO 2008/058417 A1, Figur 2 dargestellte Ausführungsform umfasst eine geschlossene Rückkopplungsschleife um die arterielle Sauerstoffsättigung $SpO_2$ während der Beatmung zu regeln. Eine Steuerung liefert ein Signal an einen Aktuator, um den Anteil des eingeatmeten Sauerstoffs ($FiO_2$) zu steuern. Die Steuerung benutzt nicht nur den Unterschied zwischen dem Ziel- und dem Ist-$S_pO_2$, sondern berücksichtigt auch die Geschwindigkeit und Beschleunigung von $SpO_2$-Änderungen und Antwortzeiten.

Es ist Aufgabe der Erfindung, ein Beatmungsgerät sowie ein Steuerverfahren hierfür anzugeben, bei dem die Gefahr der Lungenschädigung gering ist.

Diese Aufgabe wird durch die Lehre der unabhängigen Ansprüche gelöst.

Bevorzugte Ausführungsformen der Erfindung sind Gegenstand der Unteransprüche.

| Abkürzung | für |
|---|---|
| BIPAP | Bi Positive Airway Pressure |
| BU | Backup |
| CPAP | Continuous Positive Airway Pressure |
| EFE | effusive exhalation |
| FCBU | Frequency Controlled Back-Up |
| $FiO_2$ | Sauerstoffanteil im inspiriertem Atemgas |
| IP | inspiratory pressure |
| IPPV | Intermittent Positive Pressure Ventilation |
| NIV | non invasive Ventilation |

(fortgesetzt)

| Abkürzung | für |
|---|---|
| PAV | Proportional Assist Ventilation |
| PEEP | Positive End-Exspiratory Pressure |
| S-IPPV | Synchronized IPPV |
| $SpO_2$ | Sauerstoffsättigung im Blut |
| SNIPPV | Synchronized non-invasive IPPV |
| VCV | Volume Controlled Ventilation |
| Tabelle 1: Abkürzungen | |

Im Folgenden werden bevorzugte Ausführungsformen der Erfindung unter Bezugnahme auf die beiliegenden Zeichnungen näher erläutert. Dabei zeigen:

Fig. 1 ein erfindungsgemäßes Beatmungssystem;

Fig. 2 eine optionale Regelschleife zur Steuerung des Sauerstoffanteils in der Atemluft;

Fig. 3 ein Flussdiagramm für die erfindungsgemäße Regelung bei invasiver Beatmung; und

Fig. 4 bis 6 ein Flussdiagramm für die erfindungsgemäße Regelung bei nicht-invasiver Beatmung.

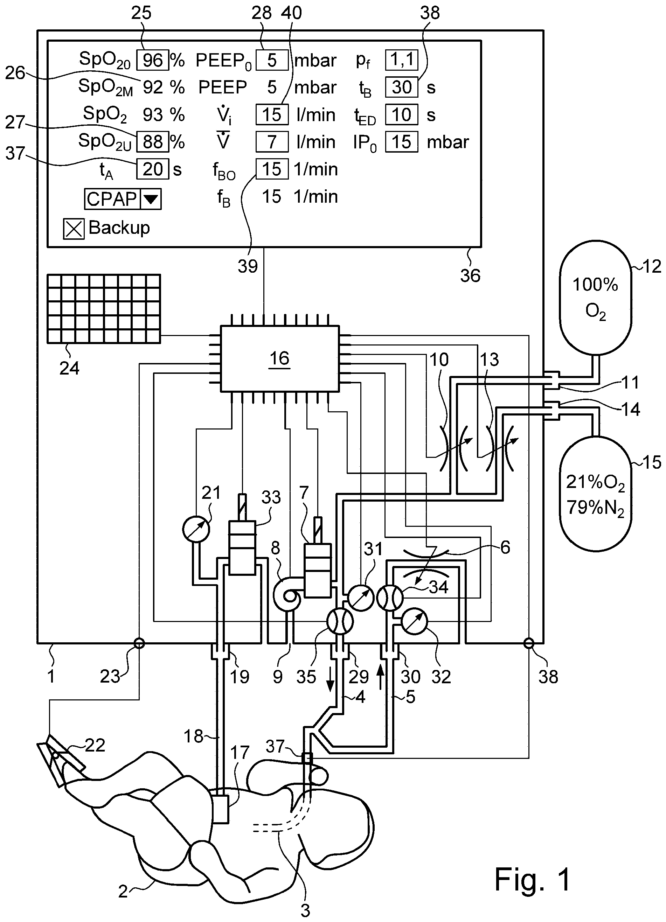

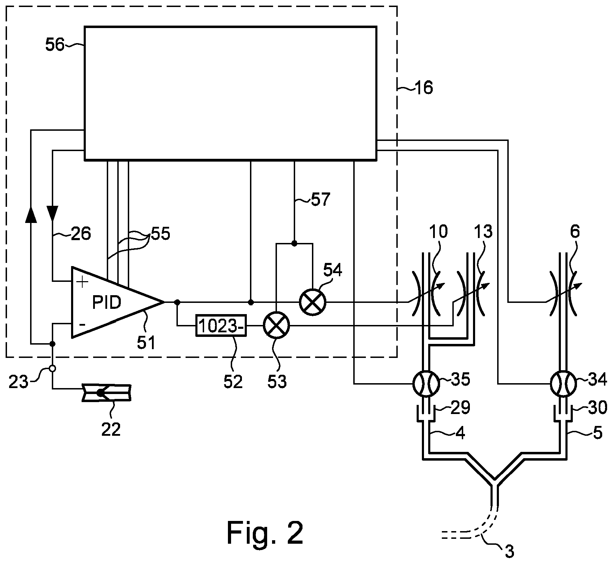

Figur 1 zeigt ein erfindungsgemäßes Beatmungsgerät 1, mit dem ein neonataler oder pädiatrischer Patient 2 über einen Endotrachealschlauch 3 beatmet wird. Alternativ zum Endotrachealschlauch 3 kann auch eine Beatmungsmaske eingesetzt werden. In Figuren 1 und 2 sind elektrische Leitungen durch eine Linie und pneumatische Leitungen durch eine Doppellinie dargestellt. Die Beatmung erfolgt über zwei Beatmungsschläuche 4 und 5, wobei über den Beatmungsschlauch 4 frische Atemluft zum Einatmen dem Frühgeborenen 2 zugeführt wird und über den Beatmungsschlauch 5 ausgeatmete Luft abgeführt wird. Hierdurch wird verhindert, dass sich Kohlendioxid in den Beatmungsschläuchen anreichert.

Falls das Beatmungsgerät 1 ein Intensivrespirator ist, ist er üblicherweise an eine Krankenhausgasversorgung angeschlossen, die beispielhaft als Sauerstoffflasche 12 und Gasflasche 15 dargestellt ist. Die Gasflaschen können aber auch real sein und neben oder, bei geeigneter Bauweise, im Beatmungsgerät untergebracht sein. Sauerstoffflasche 12 ist an Anschluss 11 und Gasflasche 15 an Anschluss 14 angeschlossen. Ein Intensivrespirator wird also üblicherweise über den Anschluss 11 mit medizinischem Sauerstoff und über den Anschluss 14 mit medizinischer Druckluft versorgt. Luft gelangt vom Anschluss 14 über ein Proportionalventil 13 zum Anschluss 29. Zusätzlich oder alternativ zu Gasflasche 15 und Anschluss 14 kann über ein Gebläse 8 Frischluft über eine Öffnung 9 im Beatmungsgerät 1 angesaugt und über ein Ventil 7 über einen Anschluss 29 in den Beatmungsschlauch 4 geblasen werden. Die Luft aus der Gasflasche 15 oder der Krankenhausluftversorgung ist steril. Dies ist bei der durch die Öffnung 9 angesaugten Umgebungsluft schwieriger sicherzustellen. Die Sauerstoffflasche 12 ist optional. Sie dient dazu, den Sauerstoffanteil des Atemgases $FiO_2$ definiert anzuheben. Der Sauerstoff wird über den Anschluss 11 und das Proportionalventil 10 über den Anschluss 29 dem Beatmungsschlauch 4 zugeführt. Der Sauerstoffgehalt in der Sauerstoffflasche 12 liegt im Allgemeinen deutlich über 21% und kann bis nahezu 100% betragen.

In anderen Ausführungsformen ist das Gebläse 8 sowie das Ventil 7 durch je ein Proportionalventil, nämlich einem Inspirationsventil und einem Exspirationsventil vor den Sensoren 34 bzw. 35 ersetzt.

Die Proportionalventile 10, 13 und 6, das Ventil 7 sowie das Gebläse 8 bzw. die alternativen Proportionalventile sind elektrisch mit der Steuerung 16 verbunden und werden von der Steuerung 16 gesteuert. Durch die Drehzahl des Gebläses 8 bzw. der Öffnungsweite des Inspirationsventils und die Stellung der Ventile 6, 7, 10 und 13 wird sichergestellt, dass während einer Inspirationsphase frische Atemluft durch den Beatmungsschlauch 4 dem Frühgeborenen 2 zugeführt und während einer Exspirationsphase ausgeatmete Luft durch den Beatmungsschlauch 5 abgeführt wird.

Damit das Beatmungsgerät 1 das Frühgeborene 2 im Hinblick auf seine Atemtätigkeit optimal unterstützen kann, muss das Beatmungsgerät 1 Eigenatmung des Frühgeborenen 2 erkennen und die Beatmung an den Bedarf des Patienten anpassen, ohne dass eine Hyper- oder Hypoventilation vorliegt. Da Frühgeborene 2 und auch Neugeborene ausgesprochene "Bauchatmer" sind, kann man die Atemtätigkeit in vorteilhafter Weise über einen am Bauch angebrachten Sensor, beispielsweise eine Grasby-Kapsel 17 erfassen, wie das in der EP 2 671 509 A1 beschrieben ist. Der Druck in der Grasby-Kapsel 17 wird über einen Schlauch 18 zum Anschluss 19 des Beatmungsgeräts 1 übertragen. Der Druck am Anschluss 19 wird über einen Drucksensor 21 in ein elektrisches Signal gewandelt, das der Steuerung 16 zugeführt wird und als Atemsignal bezeichnet werden kann. Die Steuerung 16 kalibriert die Grasby-Kapsel 17 von Zeit zu Zeit, also beispielsweise einmal in 10 Atemzügen, wobei insbesondere ein Offset neu bestimmt wird. Falls der Betrag des Offset zu groß wird, kann entweder Luft aus der Grasby-Kapsel 17 über Ventil 33 in die Umgebung abgegeben werden oder Luft aus der Umgebung über das Ventil 33 in die Grasby-Kapsel 17 gelangen. Im normalen Betrieb ist das Ventil 33 geschlossen. Die Kompression der Grasby-Kapsel 17 führt zu einem Druckanstieg, der durch den Drucksensor 21 erfasst wird. Die Füllung der Kapsel mit geschäumtem Material dient der Relaxation der Kapsel bei Entlastung also Ausatmen. Das Ventil 33 ist elektrisch mit der Steuerung 16 verbunden und wird von dieser gesteuert.

Der Druck in der Grasby-Kapsel 17 und damit auch das vom Drucksensor 21 gelieferte elektrisches Signal steigt monoton mit dem Lungenvolumen näherungsweise linear an. Das nichtlinearste Element dürfte die Grasby-Kapsel 17 selbst sein. Das Inspirationsvolumen während eines Atemzugs ergibt sich als Kalibrierungsfaktor mal Differenz zwischen dem maximalen Druck am Ende der Insufflation und dem minimalen Druck am Anfang der Insufflation. Das Exspirationsvolumen während eines Atemzugs ergibt sich als Kalibrierungsfaktor mal Differenz zwischen dem minimalen Druck am Ende der Exhalation und dem maximalen Druck am Anfang der Exhalation. Da die erfindungsgemäße Steuerung lediglich auf Quotienten von Exspirationsvolumen geteilt durch Inspirationsvolumen abstellt, braucht der Kalibrierungsfaktor nicht bestimmt zu werden. Insofern kann man die Differenz zwischen dem Druck zur Zeit t in der Grasby-Kapsel 17 und dem minimalen Druck während der entsprechenden Atemphase als Lungenvolumensignal zur Zeit t bezeichnen.

Ein Lungenvolumensignal lässt sich alternativ auch aus dem ein- und ausgeatmetem Luftfluss $\dot{V}$ bestimmen. Um diese Luftflüsse zu bestimmen können die Flusssensoren 35 bzw. 34 vorgesehen sein, die mit der Steuerung 16 elektrisch verbunden sind, um der Steuerung die Flussmesswerte mitzuteilen. Alternativ oder ergänzend kann sich auch ein patientennaher Flusssensor an dem Patientenanschluss 37 befinden, der elektrisch oder pneumatisch über den Anschluss 38 mit der Steuerung 16 verbunden ist und den Fluss vorzeichenrichtig misst. Um den Atemfluss im Mund und der Luftröhre des Frühgeborenen 2 zu berechnen, wird das Signal des Flusssensors 34 vom Signal des Flusssensors 35 abgezogen. Dabei wird der Fluss in Richtung dem Frühgeborenen 2 positiv und vom Frühgeborenen weg negativ angesehen. Der Atemfluss im Mund und der Luftröhre des Frühgeborenen 2 kann ebenfalls als Atemsignal angesehen werden. Um aus dem Atemfluss im Mund ein Inspirations- und Exspirationsvolumen während eines Atemzugs zu bestimmen, muss man die einzelnen Messwerte des Gesamtflusses während der Inspirations- bzw. Exspirationsphase aufsummieren. Um aus dem Gesamtfluss ein Lungenvolumensignal zum Zeitpunkt t zu erhalten addiert man alle Gesamtflussmesswerte ab einem Zeitpunkt to bis zum Zeitpunkt t. to wird sinnvollerweise auf den Anfang einer Inspirationsphase gelegt.

Überwacht man die Summe der Flusswerte während einer Inspirationsphase, kann man die Inspirationsphase beenden, wenn die Summe einen Schwellenwert, z.B. 0,5 l überschreitet, und so ein bestimmtes Inspirationsvolumen garantieren. Tatsächlich liegen die Flusswerte zunächst als Integer-Werte, die nicht in l/s geeicht sind in der Steuerung vor. Um Rechenzeit zu sparen, ist es sinnvoll, den Schwellenwert in l unter Berücksichtigung der Samplingrate von 100 Hz bis 1 kHz in einen Integer-Grenzwert für die Summe der Flusswerte umzurechnen.

Schließlich sind optional zwei Drucksensoren 31 und 32 vorgesehen, um den Luftdruck nahe bei den Anschlüssen 29 bzw. 30 zu messen. In Kenntnis der von den Flusssensoren 34 und 35 gemessenen Luftflüsse lässt sich der Luftdruck überall in den Beatmungsschläuchen 4 und 5 und dem Endotrachealschlauch 3 berechnen. Alternativ kann ein Drucksensor den Druck an dem Patientenanschluss 37 erfassen.

Wichtig für die Regelung ist noch, dass die Steuerung 16 über ein Oxymeter 22 die Sauerstoffsättigung $SpO_2$ im Blut des Frühgeborenen 2 messen kann. Das Oxymeter 22 ist über den Anschluss 23 und ein Kabel elektrisch mit der Steuerung 16 verbunden.

Das Beatmungsgerät 1 umfasst schließlich eine Benutzerschnittstelle mit einem Eingabesystem 24 wie z. B. eine Tastatur für die Eingabe von Beatmungsparametern sowie eine Anzeige 36 zum Anzeigen von Beatmungsparametern sowie von Messwerten. Sowohl das Eingabesystem 24 als auch die Anzeige 36 sind elektrisch mit der Steuerung 16 verbunden. Insbesondere kann ein Benutzer einen oberen Grenzwert $SpO_{2O}$ 25 sowie einen unteren Grenzwert $SpO_{2U}$ 27 für die Sauerstoffsättigung $SpO_2$ eingeben. Hieraus berechnet die Steuerung 16 das arithmetische Mittel $SpO_{2M}$, das als Sättigungszielwert 26 bezeichnet wird. Der Sättigungszielwert 26 kann, muss aber nicht angezeigt werden. In einer anderen Ausführungsform kann der Sättigungszielwert 26 auch vom Benutzer eingegeben und damit unabhängig von dem unteren und oberen Grenzwert $SpO_{2U}$ 27 bzw. $SpO_{2U}$ 27 gewählt werden. Beispielhaft für andere Beatmungsparametern ist der von der Steuerung gewählte Druck am Ende der Exspiration PEEP 28, der eingestellte Druck am Ende der Exspiration $PEEP_0$, der Inspirationsfluss $\dot{V}_i$, die eingestellte Beatmungsfrequenz $f_{B0}$, die Beatmungsfrequenz $f_B$, das Atemminutenvolumen $\overline{\dot{V}}$, die EFE-Ereignisdauer (EFE: effusive exhalation, deutsch etwa: überschwängliche Ausatmung) $T_{E0}$, den Druckfaktor $p_{fr}$, die Apnoe-Dauer $t_A$ und die Mindestverweildauer $t_B$ angezeigt. Fakultativ kann auch der Standarddruck während der Inspiration IPo eingegeben werden.

Die Steuerung 16 kann einen Prozessor zur Ausführung von Software, Arbeitsspeicher, einen nicht flüchtigen Speicher, Analog-Digital-Wandler, Digital-Analog-Wandler sowie Leistungselektronik aufweisen. Der exakte elektrische Aufbau der Steuerung 16 kann von einem Fachmann entworfen werden und liegt im Rahmen seines Fachwissens.

Anhand des Blockschaltbilds in Figur 2 wird eine optionale Regelungsschleife zur Steuerung des Sauerstoffanteils im inspirierten Atemgases $FiO_2$ erläutert. Die Proportionalventile 6, 10 und 13, die Flusssensoren 35 und 34, die Beatmungsschläuche 4 und 5, der Endotrachealschlauch 3, das Oxymeter 22 sowie die Anschlüsse 23, 29 und 30 wurden bereits bei Figur 1 beschrieben. Die Steuerung 16 ist in Figur 2 detaillierter dargestellt. Die Steuerung 16 enthält insbesondere einen PID-Regler 51, einen Subtrahierer 52, Multiplizierer 53 und 54 sowie eine Atemzugsteuerung 56. Obwohl der PID-Regler 51, der Subtrahierer 52 sowie die Multiplizierer 53 und 54 als Bauelemente dargestellt sind, ist Fachleuten klar, dass in modernen Steuerungen diese Funktionalitäten in Software implementiert sind. Der PID-Regler 51 liefert an seinem Ausgang einen Wert zwischen einem Minimalwert beispielsweise 0 und einen Maximalwert von beispielsweise 1023. Der Subtrahierer 52 liefert einen zweiten Wert, der addiert mit dem Wert des PID-Reglers gerade den Maximalwert ergibt. Der Wert des PID-Reglers und der zweite Wert werden in den Multiplikatoren 53 bzw. 54 je mit einem Inspirationswert 57 multipliziert. Die Ergebnisse der Multiplikatoren 53 und 54 steuern die Proportionalventile 13 bzw. 10. So lässt sich der Gesamtinspirationsfluss, der über den Flusssensor 31 gemessen wird, über Inspirationswert 57 weitgehend unabhängig vom Sauerstoffanteil $FiO_2$ einstellen. Umgekehrt lässt sich die Stellgröße, nämlich der Sauerstoffanteil $FiO_2$ weitgehend unabhängig vom Inspirationsfluss, einstellen.

Die Zielgröße des PID Reglers 51 ist der Sättigungszielwert $SpO_{2M}$ 26 und wird dem PID-Regler 51 über den +-Eingang zugeführt. Die Istgröße der Sauerstoffsättigung $SpO_2$ wird dem --Eingang des PID-Reglers zugeführt.

Im Betrieb ist das Frühgeborene 2 Teil der Regelstrecke. Der Zusammenhang zwischen dem Sauerstoffanteil $FiO_2$ und der Sauerstoffsättigung $SpO_2$ ist nichtlinear. Deshalb ist es sinnvoll, die Regelparameter 55 des PID-Reglers 51, also die Verstärkungsfaktoren für den Proportional-, Integral- und Differenzialanteil beispielsweise anhand der eingestellten Grenzen $SpO_{2O}$ 25 und $SpO_{2U}$ 28 sowie der Richtung der Änderung der Sauerstoffsättigung $SpO_2$ zu ändern. Der Zusammenhang zwischen dem Sauerstoffanteil $FiO_2$ und der Sauerstoffsättigung $SpO_2$ hängt auch vom Zustand des Frühgeborenen ab, also beispielsweise davon, ob das Frühgeborene schläft oder wach ist. Deshalb können weitere Parameter wie das Atemzugvolumen bei PAV oder die Sauerstoffsättigung $SpO_2$ selbst berücksichtigt werden.

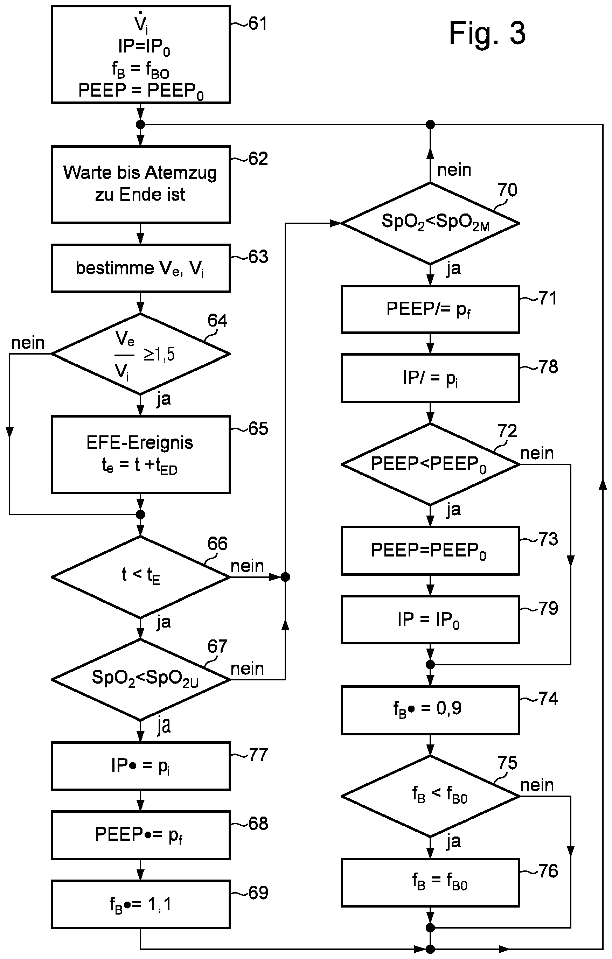

Figur 3 zeigt ein Flussdiagramm, das wichtige Schritte in der Atemzugsteuerung 56 für eine invasive Beatmungsform mit Überwachung des Tidalvolumen, beispielsweise IPPV darstellt. Im Schritt 61 wird der Inspirationsfluss $\dot{V}_i$ eingestellt, die Beatmungsfrequenz $f_B$ gleich der eingestellten Beatmungsfrequenz $f_{B0}$ gesetzt und der Druck am Ende der Exspiration (PEE-Druck, PEEP) gleich dem eingestellten Druck am Ende der Exspiration ($PEEP_0$) gesetzt. Fakultativ kann auch der Druck der Inspiration IP auf den Standardwert $IP_0$ gesetzt werden. Die in Figur 3 dargestellten Schritte werden einmal nach jedem Atemzug durchlaufen. Deshalb wird im Schritt 62 gewartet, bis ein Atemzug zu Ende ist. Gemäß der üblichen Konvention beginnt ein Atemzug mit der Inspiration und endet mit der Exspiration. Für die Erfindung könnte man einen Atemzug aber auch mit der Exspiration beginnen und mit der Inspiration enden lassen. Die Kriterien, wann Exspiration und Inspiration enden, ergeben sich aus der Beatmungsform, beispielsweise IPPV.

In Schritt 63 werden das Inspirationsvolumen $V_i$ und das Exspirationsvolumen $V_e$ bestimmt. "Bestimmen" in Schritt 63 kann lediglich Auslesen bedeuten. Insbesondere bei der IPPV kann das Sollinspirationsvolumen aus dem Atemminutenvolumen $\overline{\dot{V}}$ und der Beatmungsfrequenz $f_B$ berechnet werden. Sinnvollerweise werden auch während der Exspiration die einzelnen Atemflussmesswerte aufaddiert, so dass am Ende der Exspiration das Exspirationsvolumen $V_e$ ausgelesen werden kann.

Im Vergleich 64 wird das Exspirationsvolumen mit dem Inspirationsvolumen verglichen. Übersteigt das Exspirationsvolumen das Inspirationsvolumen um wenigstens 50 %, ist also $V_e/V_i >= 1{,}5$, so wird in Schritt 65 ein EFE-Ereignis gespeichert, beispielsweise, in dem eine Endzeit $t_E$ als Summe aus der aktuellen Zeit t plus einer vorgegebenen oder einstellbaren EFE-Ereignisdauer $t_{ED}$ berechnet und gespeichert wird. In einer anderen Ausführungsform kann ein von 50% abweichender Schwellenwert vom Bedienpersonal eingestellt werden. $t_{ED}$ beträgt mindestens einen Atemzug und ist sinnvollerweise länger und vom Benutzer einstellbar. Der Ablauf der EFE-Ereignisdauer $t_{ED}$ wird im Vergleich 66 überprüft. Dies geschieht je nach dem Ergebnis im Vergleich 64 unmittelbar nach Vergleich 64 oder Schritt 65. In Vergleich 67 wird geprüft, ob nach dem EFE-Ereignis innerhalb der EFE-Ereignisdauer $t_{ED}$ die gemessene Sauerstoffsättigung $SpO_2$ unter den eingestellten unteren Grenzwert $SpO_{2U}$ fällt. Ist das der Fall, so wird der PEEP in Schritt 68 um einen vorher vom Anwender eingestellten Druckfaktor $p_{fr}$, beispielsweise 10%, erhöht. Fakultativ kann kurz davor oder danach, also etwa zur gleichen Zeit in Schritt 77 der Druck der Inspiration IP um einen Faktor $p_i$ angehoben werden, um die Menge an ausgetauschtem Gas konstant zu halten. Anstelle eines Faktors kann der Anwender auch einen festen Druckwert einstellen, um den der PEE-Druck erhöht werden muss. Dies gilt auch für den Druck der Inspiration. Die Reihenfolge der Vergleiche 66 und 67 kann auch vertauscht werden. Um die Menge an ausgetauschtem Gas in dieser adaptierten Phase der Beatmung konstant zu halten kann die Beatmungsfrequenz $f_B$ in Schritt 69 erhöht werden.

Zum Schritt 70 gelangt man, wenn die Vergleiche 66 oder 67 nein liefern. In Vergleich 70 wird geprüft, ob die Sauerstoffsättigung $SpO_2$ den Sättigungszielwert $SpO_{2M}$ überschreitet. Falls dies nicht der Fall ist, wird in Schritt 62 auf das Ende des nächsten Atemzugs gewartet. Überschreitet die Sauerstoffsättigung $SpO_2$ den Sättigungszielwert $SpO_{2M}$, so wird der PEEP schrittweise auf den ursprünglichen Wert zurückgeführt, wobei in jedem Schritt um den Druckfaktor $p_{fl}$ dividiert wird. Die Druckfaktoren $p_{fr}$ und $p_{fl}$ können gleich sein, aber sinnvollerweise ist $p_{fl}$ kleiner als $p_{fr}$, so dass die Absenkung des PEEP langsamer als die vorherige Anhebung erfolgt. Einer dieser Schritte ist in Schritt 71 dargestellt. Wenn der Druck der Inspiration IP in Schritt 77 angehoben wurde, wird er in Schritt 78 in gleicher Weise, also entweder durch Division durch $p_i$ oder subtrahieren einer festen Druckdifferenz abgesenkt. In Vergleich 72 wird geprüft, ob der PEEP schon unter den eingestellten Druck am Ende der Exspiration $PEEP_0$ gefallen ist. Ist dies der Fall, wird in Zuweisung 73 der PEEP auf den $PEEP_0$ gesetzt. Wurde die Beatmungsfrequenz $f_B$ bei der Anpassung des PEEP ebenfalls geändert, so wird auch diese auf ähnliche Weise in den Zuweisungen 74 und 76 sowie im Vergleich 75 wieder auf die eingestellte Beatmungsfrequenz $f_{B0}$ zurückgeführt. Wenn der Druck der Inspiration IP in Schritt 77 angehoben wurde, wird er in Schritt 79 gleich dem Standardwert IPo gesetzt.

Im Anschluss an die Zuweisungen 69 oder 76 oder den Vergleich 75 wird in Schritt 62 auf das Ende des nächsten Atemzugs gewartet.

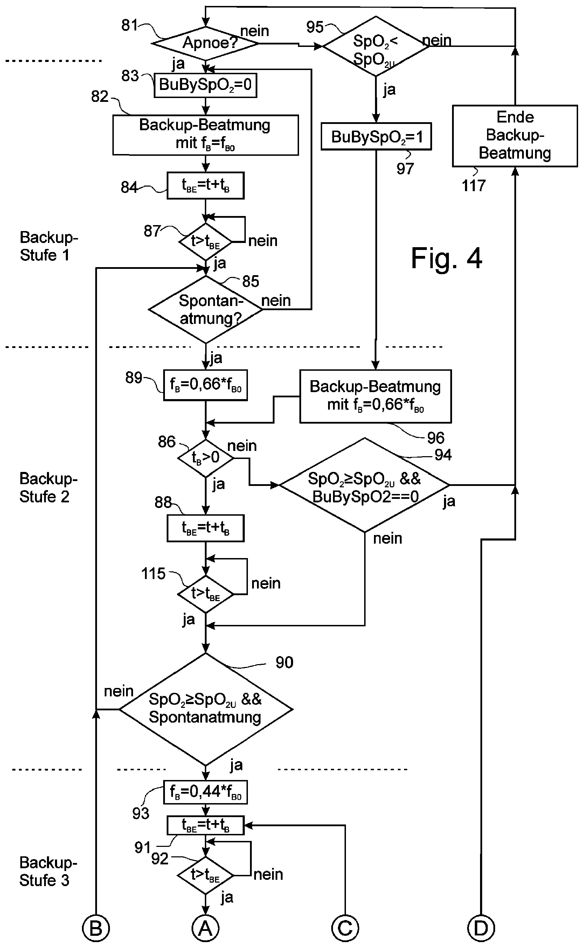

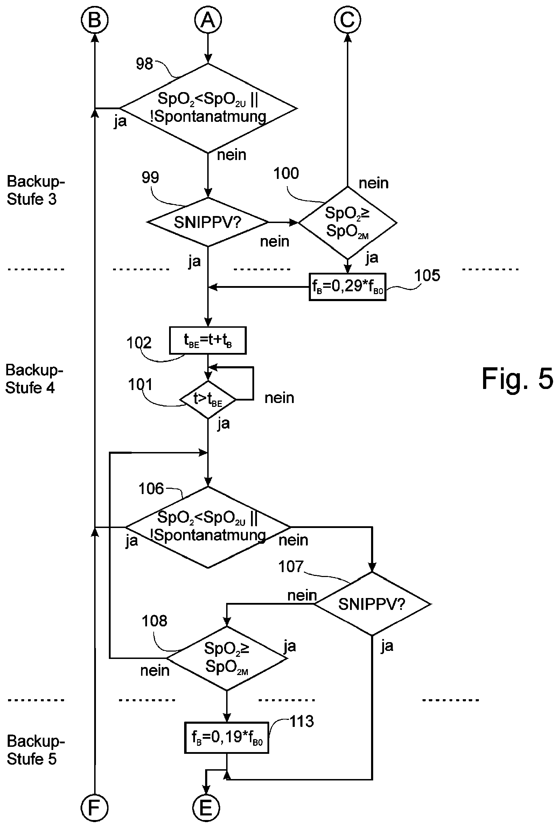

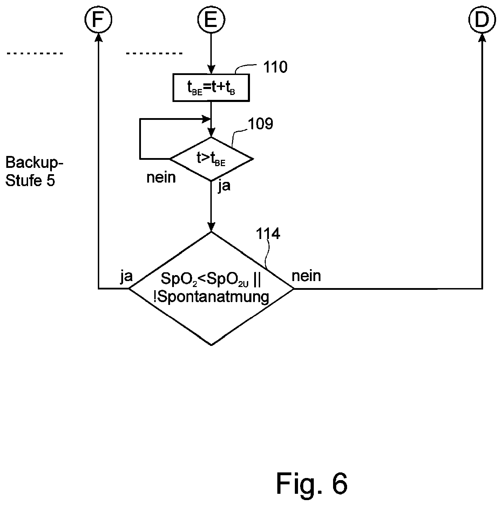

Figuren 4 bis 6 zeigen ein Flussdiagramm für die erfindungsgemäße Regelung bei einer nicht-invasiver Beatmungsform mit eingestellter Apnoe-Dauer $t_A$, also beispielsweise CPAP oder SNIPPV. Hier wird die Intensität der Beatmung, also insbesondere die Beatmungsfrequenz $f_B$, anhand des Abfalls der Sauerstoffsättigung gesteuert. Für die Beatmungsfrequenz $f_B$ gibt es fünf Backup-Stufen. Die Beatmungsfrequenz $f_B$ auf Stufe 1 ist die eingestellte Beatmungsfrequenz $f_{B0}$. Von Backup-Stufe zu Backup-Stufe nimmt die Beatmungsfrequenz um 2/3 ab. Die Beatmungsfrequenzen für die Stufen 2 und 3 betragen also 2/3 (etwa 66%) $f_{B0}$ bzw. 4/9 (ca. 44%) $f_{B0}$. Auf jeder Stufe gibt es eine einstellbare Mindestverweildauer $t_B$, die typischerweise 30s beträgt. In einer Ausführungsform kann die Mindestverweildauer aus 4 Werten, nämlich 0s, 10s, 30s oder 60s ausgewählt werden. Ist diese Mindestverweildauer auf 0 eingestellt, dann wird die Backup-Beatmung beendet, sobald der Grund der Backup-Beatmung nicht mehr besteht. Eine eingestellte Verweildauer gößer 0 wird als FCBU (Frequency Controlled Back-Up, deutsch: frequenzgesteuertes Backup) bezeichnet. Unter SNIPPV wird die Beatmungsfrequenz nicht unter 4/9 $f_{BO}$ und unter CPAP oder nCPAP nicht unter 16/81 (ca. 20%) $f_{B0}$ abgesenkt.

Falls in Schritt 81 innerhalb der Apnoe-Dauer $t_A$ kein Atemzug erkannt wurde, also eine Apnoe vorliegt, wird in Schritt 83 die Variable $BuBySpO_2$ auf 0 gesetzt und so der Grund für den Start der Backup-Beatmung gespeichert. In Schritt 82 wird die Backup-Beatmung mit der eingestellten Beatmungsfrequenz $f_{B0}$ in Backup-Stufe 1 gestartet. Als Backup-Beatmung wird eine synchronisierte Beatmungsform, z.B. SNIPPV, verwendet. In Schritt 84 wird das frühestmögliche Ende der Stufe $t_{BE}$ als Summe aus der aktuellen Zeit t und der Mindestverweildauer $t_B$ berechnet. In Schritt 87 wird das Ende der Mindestverweildauer abgewartet.

Falls in Schritt 85 Spontanatmung erkannt wird, wird bei aktiviertem FCBU (Vergleich 86) die volle eingestellte Beatmungsfrequenz $f_{B0}$ um 1/3 in Schritt 89 auf etwa 66% reduziert, also in Backup-Stufe 2 fortgesetzt. Falls in Schritt 85 keine Spontanatmung erkannt wird, wird die Backup-Beatmung mit der Frequenz $f_{B0}$ in den Schritten 83 und 82 fortgesetzt. Hieraus ergibt sich, dass die Dauer der Backup-Stufe 1 $n*t_{BE}$ beträgt, wenn $t_{BE}>0$, wobei n eine ganze Zahl ist. Wurde in Schritt 85 eine Spontanatmung festgestellt, so wird die Backup-Beatmung in Stufe 2 fortgesetzt und in Schritt 89 die neue Beatmungsfrequenz berechnet und eingestellt. Bei aktiviertem FCBU, also $t_B>0$, wird nach Vergleich 86, in Schritt 88 neuerlich die Zeit $t_{BE}$ bestimmt. In Schritt 115 wird deren Ablauf abgewartet. Ist FCBU in Vergleich 86 nicht aktiviert und liegt die Sauerstoffsättigung in Vergleich 94 über dem unteren Grenzwert $SpO_{2U}$ und wurde die Backup-Beatmung wegen einer Apnoe in Schritt 81 gestartet, ist also $BuBySpO_2==0$, so wird zur Beendigung der Backup-Beatmung 117 gesprungen. Wie in vielen Programmiersprachen steht dabei && für einen logischen Und-Operator und == für einen Vergleichsoperator, der auf Gleichheit prüft.

Andernfalls wird im Vergleich 90 geprüft, ob die Sauerstoffsättigung $SpO_2$ über dem unteren Grenzwert der Sauerstoffsättigung $SpO_{2U}$ liegt und weiterhin Spontanatmung vorherrscht. Ist dies der Fall, wird die Backup-Beatmung in Schritt 93 in Backup-Stufe 3 mit einer Beatmungsfrequenz $f_B$ von 4/9 der eingestellten Beatmungsfrequenz $f_{B0}$ fortgesetzt. Dann wird in Schritt 91 das Ende der Mindestverweildauer $t_{BE}$ berechnet, in Schritt 92 die Mindestverweildauer abgewartet.

Liegt die Sauerstoffsättigung $SpO_2$ bei Vergleich 90 unter dem unteren Grenzwert der Sauerstoffsättigung $SpO_{2U}$, ist in Schritt 85 Spontanatmung erkannt worden und ist in Schritt 86 FCBU aktiviert, beginnt die Mindestverweildauer $t_B$ in Schritt 88 erneut. Dies wird dadurch erreicht, dass in Schritt 88 das Ende $t_{BE}$ neu berechnet, also in die Zukunft verschoben wird.

In Vergleich 98 wird ähnlich wie in Vergleich 90 auf das Vorhandensein von Spontanatmung oder Unterschreiten des Grenzwertes $SpO_{2U}$ durch die Sauerstoffsättigung $SpO_2$ geprüft. Wie in vielen Programmiersprachen steht dabei "∥" für einen logischen oder-Operator und "!" für eine Negation. Liegt in Vergleich 98 keine Spontanatmung vor oder ist der Grenzwert $SpO_{2U}$ unterschritten, so wird zu Vergleich 85 gewechselt. Eine fehlende Spontanatmung in Vergleich 85 führt zum Eintritt in die Backup-Stufe 1 über die Schritte 83 und 82.

Unter SNIPPV, was in Vergleich 99 überprüft wird, wird ohne Frequenzwechsel in die nächste Backup-Stufe 4 gewechselt. Zeigte Vergleich 99, dass nicht SNIPPV, sondern CPAP als Beatmungsform gewählt war, so wird in Vergleich 100 geprüft, ob die Sauerstoffsättigung den Sättigungszielwert $SpO_{2M}$ erreicht hat. Ist dies nicht der Fall, verbleibt die Steuerung in Backup-Stufe 3 und fährt mit der Berechnung des neuen frühesten Endes $t_{BE}$ in Schritt 91 fort.

Hat die Sauerstoffsättigung $SpO_2$ in Vergleich 100 den Sättigungszielwert $SpO_{2M}$ erreicht, so wird auch unter CPAP in die Backup-Stufe 4 gewechselt und in Schritt 105 die neue Backup-Frequenz $f_B=0.29 \times f_{B0}$ berechnet und eingestellt. In Stufe 4 wird dann das neue früheste Ende $t_{BE}$ in Schritt 102 berechnet, das Ende $t_{BE}$ in Schritt 101 abgewartet. In Vergleich 106 wird erneut geprüft, ob Spontanatmung vorhanden ist und die Sauerstoffsättigung nicht unter den Grenzwert $SpO_{2U}$ gefallen ist. Sollte eine der beiden Bedingungen nicht erfüllt sein, so wird, wie zuvor nach Vergleich 98, zu Vergleich 85 zurückgesprungen.

Ist in Vergleich 106 Spontanatmung vorhanden und die Sauerstoffsättigung $SpO_2$ mindestens gleich dem Grenzwert $SpO_{2U}$, so wird im Falle der Beatmungsform SNIPPV, was in Vergleich 107 geprüft wird, ohne Frequenzwechsel in die Backup-Stufe 5 gewechselt. Ist die Beatmungsform nicht SNIPPV, so wird in Vergleich 108 zusätzlich geprüft, ob die Sauerstoffsättigung $SpO_2$ den Sättigungszielwert $SpO_{2M}$ erreicht hat. Ist dies nicht der Fall, dann verbleibt das Backup in Backup-Stufe 4, wobei mit Vergleich 106 fortgesetzt wird. Wurde im Vergleich 108 festgestellt, dass die Sauerstoffsättigung $SpO_2$ den Sättigungszielwert $SpO_{2M}$ erreicht hat, so wird in die Backup-Stufe 5 gewechselt und in Schritt 113 die Beatmungsfrequenz $f_B$ auf $0.19 \times f_{B0}$ abgesenkt. Unabhängig von CPAP oder SNIPPV wird in Backup-Stufe 5 im Schritt 110 wird das früheste Ende $t_{BE}$ der Stufe 5 berechnet und in Schritt 109 dieses Ende abgewartet. Im Vergleich 114 wird wiederum geprüft, ob Spontanatmung vorhanden und die Sauerstoffsättigung $SpO_2$ mindestens gleich dem Grenzwert $SpO_{2U}$ ist. Ist keine Spontanatmung vorhanden oder die Sauerstoffsättigung unter den Grenzwert $SpO_{2U}$ gefallen, so wird zu Schritt 85 gewechselt. Ist Spontanatmung vorhanden und die Sauerstoffsättigung mindestens gleich dem Grenzwert $SpO_{2U}$ so wird das Backup in Schritt 117 beendet.

Falls in Schritt 81 keine Apnoe erkannt wurde, also Spontanatmung vorhanden war oder die Spontanatmung für weniger als die der Apnoe-Dauer $t_A$ aussetzte, wird in Schritt 95 geprüft, ob die Sauerstoffsättigung $SpO_2$ unter dem unteren Grenzwert der Sauerstoffsättigung $SpO_{2U}$ liegt. Falls dies auch nicht der Fall ist, kann die Atemzugsteuerung 56 warten und danach in Schritt 81 wieder prüfen, ob eine Apnoe vorliegt. Falls die Sauerstoffsättigung $SpO_2$ in Vergleich 95 unter dem unteren Grenzwert der Sauerstoffsättigung $SpO_{2U}$ liegt, wird die Backup-Beatmung in Schritt 96 mit einer Beatmungsfrequenz $f_B$ von 2/3, also etwa 66%, der eingestellten Beatmungsfrequenz $f_{B0}$ in der Backup-Stufe 2 gestartet. Zuvor wird in Schritt 97 der Einstieg in das Backup über einen Sättigungsabfall, also über Schritt 95, gespeichert, indem die Variable $BuBySpO_2 =1$ gesetzt wird, um den eventuellen vorzeitigen Ausstieg im Vergleich 94 zu verhindern. Im weiteren Verlauf ist die Abfolge der Prozessschritte identisch mit der bei einer Backup-Beatmung, die durch eine Apnoe im Vergleich 81 ausgelöst wurde.

Auch wenn das in den Fig. 4 bis 6 nicht explizit dargestellt ist, läuft die Apnoe-Detektion in Schritt 81 auch während der Backup-Beatmung mit. Sollte in den höheren Backup-Stufen eine Apnoe auftreten, wird zu den Schritten 83 und 82 zur Backup-Stufe 1 gewechselt. Dies kann insbesondere in den höheren Backup-Stufen ab Stufe 3 vorkommen.

## Patentansprüche

1. Beatmungsgerät zur Beatmung eines Patienten mit:

    einem Beatmungsschlauchanschluss (29, 30) zum Anschließen eines Beatmungsschlauchs (4, 5);
    einem Aktuator (8, 10, 13), der pneumatisch mit dem Beatmungsschlauchanschluss (29, 30) verbunden ist, zum Abgeben von Luft über den Beatmungsschlauchanschluss (29, 30) an einen Patienten (2);
    einen Oxymeteranschluss (23) zum Liefern eines Sauerstoffsättigungssignals;
    einer digitalen, programmierbaren Steuerung (16), die elektrisch mit dem Oxymeteranschluss (23) verbunden ist, um der digitalen, programmierbaren Steuerung (16) das Sauerstoffsättigungssignal zuzuführen, wobei die digitale, programmierbare Steuerung (16) programmiert ist,

    • um die Sauerstoffsättigung aus dem Sauerstoffsättigungssignal zu bestimmen, und
    • um zu bestimmen, ob die Sauerstoffsättigung einen unteren Grenzwert (27) unterschreitet (67) und um den Druck am Ende der Exspiration und die Atemfrequenz zu erhöhen (68, 69),

    **dadurch gekennzeichnet, dass**
    das Beatmungsgerät (1) ferner ein Ventil (13) und einen Sauerstoffanschluss (14) für ein Gas oder Gasgemisch (15) mit mehr als 21 Vol% Sauerstoff aufweist, wobei ein Einlass des Ventils (13) pneumatisch mit dem Sauerstoffanschluss (14), ein Auslass des Ventils (13) pneumatisch mit dem Beatmungsschlauchanschluss (29) und ein Steueranschluss des Ventils (13) elektrisch mit der digitalen, programmierbaren Steuerung (16) verbunden ist, wobei die digitale, programmierbare Steuerung (16) ferner einen PID-Regler (51) umfasst, der das Ventil (13) so steuert, dass die Sauerstoffsättigung möglichst wenig von einem Sättigungszielwert zwischen einem oberen Grenzwert (25) und dem unteren Grenzwert (27) abweicht.

2. Beatmungsgerät gemäß Anspruch 1, **dadurch gekennzeichnet, dass** die digitale, programmierbare Steuerung (16) ferner programmiert ist, um mit der Erhöhung des Drucks am Ende der Exspiration ebenfalls den Druck der Inspiration anzuheben.

3. Beatmungsgerät gemäß einem der vorausgehenden Ansprüche, ferner **gekennzeichnet durch**
   einen Atemsensoranschluss (19) für einen Atemsensor (17, 34, 35), zum Liefern eines Atemsignals, wobei die digitale, programmierbare Steuerung (16) elektrisch mit dem Atemsensoranschluss (19) verbunden ist, wobei der digitalen Steuerung (16) das Atemsignal zugeführt wird, wobei die digitale, programmierbare Steuerung (16) programmiert ist,

   • um aus dem Atemsignal ein Lungenvolumensignal zu berechnen, wobei das Lungenvolumensignal monoton mit dem Lungenvolumen des Patienten (2) ansteigt, wobei die Steuerung (16) programmiert ist, um aus dem Lungenvolumensignal für jeden Atemzug ein inhaliertes Volumen und ein exhaliertes Volumen zu berechnen, und
   • um ein EFE-Ereignis zu speichern (65), wenn das exhalierte Volumen das zugehörige inhalierte Volumen um einen einstellbaren Wert übersteigt (64) und um bei einem EFE-Ereignis den Druck am Ende der Exspiration zu erhöhen (68).

4. Beatmungsgerät gemäß Anspruch 3, ferner mit:

   einem Oxymeteranschluss (23), der mit der digitalen, programmierbaren Steuerung (16) elektrisch verbunden ist, zum Liefern eines Sauerstoffsättigungssignals;
   wobei die digitale, programmierbare Steuerung (16) ferner programmiert ist, um zu bestimmen, ob die Sauerstoffsättigung während einer Zeitspanne nach einem EFE-Ereignis einen unteren Grenzwert (27) unterschreitet (67) und um den Druck am Ende der Exspiration nur zu erhöhen (68), wenn die Sauerstoffsättigung während einer Zeitspanne nach einem EFE-Ereignis einen unteren Grenzwert (27) unterschreitet (67).

## Claims

1. Respirator for ventilating a patient, comprising:

   a respiratory tube connector (29, 30) for connecting a respiratory tube (4, 5);
   an actuator (8, 10, 13) that is pneumatically connected to the respiratory tube connector (29, 30) for delivering air to a patient (2) via the respiratory tube connector (29, 30);
   an oximeter connector (23) for returning an oxygen saturation signal;
   a digital programmable controller (16) that is electrically connected to said oximeter connector (23) for delivering the oxygen saturation signal to said digital programmable controller (16), wherein the digital programmable controller (16) is programmed

   • to calculate the oxygen saturation from the oxygen saturation signal, and
   • to determine whether the oxygen saturation falls below (67) a lower limit (27) and to increase the pressure at the end of exhalation and to increase the respiration rate (68, 69)

   **characterized in that**
   the respirator (1) further comprises a valve (13) and an oxygen connector (14) for a gas or mixture of gases (15) having more than 21 volume percent of oxygen, wherein an inlet of the valve (13) is pneumatically connected to the oxygen connector (14), an outlet of the valve (13) is pneumatically connected to the breathing respiratory tube connector (29) and a control connector of the valve (13) is electrically connected to the digital programmable controller (16), wherein the digital programmable controller (16) further comprises a PID controller (51), which controls the valve (13) so that the oxygen saturation differs as little as possible from a saturation setpoint between an upper limit (25) and a lower limit (27).

**2.** Respirator according to claim 1, wherein the digital programmable controller (16) is further programmed to increase the inspiratory pressure together with the pressure at the end of exhalation.

**3.** Respirator according to one of the preceding claims, further **characterized in**

a breathing sensor connector (19) for a breathing sensor (17, 34, 35) for providing a breathing sensor signal; wherein the digital programmable controller (16) is electrically connected to said breathing sensor connector (19), wherein the breathing signal is fed to said digital programmable controller (16) that is programmed

• to calculate a lung volume signal from said breathing signal, wherein the lung volume signal monotonically increases with the lung volume of the patient (2), wherein the digital programmable controller (16) is programmed to calculate an inhaled volume and an exhaled volume from the lung volume signal for each breath; and
• to store an EFE event (65) when the exhaled volume exceeds the associated inhaled volume by an adjustable value (64), and to increase the pressure at the end of exhalation at an EFE event (68).

**4.** Respirator according to claim 3, further comprising

an oximeter connector (23) that is electrically connected to said digital programmable controller (16) for delivering an oxygen saturation signal;
wherein the digital programmable controller (16) further is programmed to determine whether the oxygen saturation falls below (67) a lower limit (27) during a time period after an EFE event and to increase the pressure at the end of exhalation only if the oxygen saturation falls below (67) a lower limit (27) during a time period after an EFE event.

**Revendications**

**1.** Appareil respiratoire pour la respiration d'un patient avec :

un raccord de tuyau flexible respiratoire (29, 30) pour le raccordement d'un tuyau flexible respiratoire (4, 5) ;
un actionneur (8, 10, 13) relié de façon pneumatique au raccord de tuyau flexible respiratoire (29, 30) pour l'envoi d'air à un patient (2) via le raccord de tuyau flexible respiratoire (29, 30) ;
un raccord d'oxymètre (23) pour la délivrance d'un signal de détecteur de souffle ;
un élément de commande programmable numérique (16) qui est relié électriquement au raccord d'oxymètre (23) pour amener le signal de saturation en oxygène à l'élément de commande programmable numérique (16) ; et

• pour déterminer si la saturation en oxygène passe en dessous (67) d'une valeur limite inférieure (27) et
• pour accroître (68, 69) la fréquence respiratoire et la pression à la fin de l'expiration.

**caractérisé en ce que** :
l'appareil respiratoire (1) comporte en outre une soupape (13) et un raccord d'oxygène (14) pour un gaz ou un mélange gazeux (15) avec plus de 21 % du volume d'oxygène, une admission de la soupape (13) étant reliée de façon pneumatique au raccord d'oxygène (14), un échappement de la soupape (13) étant relié de façon pneumatique au raccord de tuyau flexible respiratoire (29) et un raccord de commande de la soupape (13) est relié électriquement à l'élément de commande programmable numérique (16), l'élément de commande programmable numérique (16) comprenant en outre un régulateur PID (51) commandant la soupape (13) de sorte que la saturation d'oxygène s'écarte le moins possible d'une valeur cible de saturation entre une valeur limite supérieure (25) et la valeur limite inférieure (27).

**2.** Appareil respiratoire selon la revendication 1, **caractérisé en ce que** l'élément de commande programmable numérique (16) est en outre programmé pour augmenter également la pression de l'inspiration à la fin de l'expiration avec l'augmentation de la pression.

**3.** Appareil respiratoire selon l'une quelconque des revendications précédentes, en outre **caractérisé par** :
un raccord de détecteur de souffle (19) pour un détecteur de souffler (17, 34, 35), conçu pour délivrer un signal de souffle, l'élément de commande programmable numérique (16) étant relié électriquement à un raccord de détecteur de souffle (19), le signal de souffle étant amené à l'élément de commande programmable numérique (16), l'élément de commande programmable numérique (16) étant programmé

• pour calculer, à partir du signal de souffle, un signal de volume pulmonaire, le signal de volume pulmonaire augmentant de façon monotone avec le volume pulmonaire du patient (2), l'élément de commande programmable numérique (16) étant programmé pour calculer à partir du signal de volume pulmonaire, pour chaque inspiration, un volume inhalé et un volume exhalé ; et

• pour mémoriser (65) un événement EFE lorsque le volume exhalé dépasse d'une valeur réglable (64) le volume inhalé associé et pour accroître la pression à la fin de l'expiration en cas d'événement EFE (68).

4. Appareil respiratoire selon la revendication 3, comprenant en outre :

un raccord d'oxymètre (23) qui est relié électriquement à l'élément de commande (16) pour délivrer ledit signal de saturation d'oxygène ;

l'élément de commande programmable numérique (16) étant en outre programmé pour déterminer si la saturation en oxygène passe pendant un intervalle de temps après un événement EFE en dessous (67) d'une valeur limite inférieure (27) et pour n'accroître (68) la pression à la fin de l'expiration que lorsque la saturation en oxygène passe en-dessous (67) d'une valeur limite inférieure (27) pendant un certain intervalle de temps.

Fig. 1

Fig. 2

Fig. 3

Fig. 4

Fig. 5

Ⓕ      Ⓔ                         Ⓓ

- - - - - - - - - -    - - - - - - - - -

$$t_{BE} = t + t_B$$    110

109

Backup-Stufe 5

$$t > t_{BE}$$

nein      ja

114

ja    $SpO_2 < SpO_{2U} \,||$    nein
       !Spontanatmung

# Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2011041849 A1
- EP 2091429 B1
- US 20120071729 A1
- EP 1562655 B1
- EP 1294428 B1
- WO 0247741 A2
- EP 2671509 A1
- US 2009320836 A1
- WO 2008058417 A1

**In der Beschreibung aufgeführte Nicht-Patentliteratur**

- **LAUT RATHGEBER.** Grundlagen der maschinellen Beatmung. Georg Thieme Verlag
- **YAO SUN et al.** NEONATAL WORKSTATION FOR INSPIRED OXYGEN CONTROL AND CLINICAL MONITORING. *AAAI Technical Report SS-94-01,* 1994
- Adaptive mechanical backup ventilation for preterm infants on respiratory assist modes. **VON SUSANNE HERBER-JONAT et al.** Intensive Care Med. Springer, 2006, vol. 32, 302-308